(19) **Europäisches Patentamt**
**European Patent Office**
**Office européen des brevets**

(11) **EP 3 970 785 B1**

(12) **EUROPÄISCHE PATENTSCHRIFT**

(45) Veröffentlichungstag und Bekanntmachung des Hinweises auf die Patenterteilung:
**05.03.2025 Patentblatt 2025/10**

(21) Anmeldenummer: **21205817.6**

(22) Anmeldetag: **01.07.2015**

(51) Internationale Patentklassifikation (IPC):
*A61M 60/178* (2021.01)   *A61M 60/221* (2021.01)
*A61M 60/232* (2021.01)   *A61M 60/242* (2021.01)
*A61M 60/422* (2021.01)   *A61M 60/523* (2021.01)
*A61M 60/531* (2021.01)   *A61M 60/546* (2021.01)
*A61M 60/554* (2021.01)

(52) Gemeinsame Patentklassifikation (CPC):
A61M 60/178; A61M 60/221; A61M 60/232;
A61M 60/237; A61M 60/242; A61M 60/422;
A61M 60/523; A61M 60/531; A61M 60/546;
A61M 60/554; A61M 60/816; A61M 60/857;
A61M 60/863; A61M 2205/3334

(54) **VERFAHREN UND HERZUNTERSTÜTZUNGSSYSTEM ZUR BESTIMMUNG EINES AUSLASSDRUCKS**

METHOD AND HEART SUPPORT SYSTEM FOR DETERMINING AN OUTLET PRESSURE

PROCÉDÉ ET DISPOSITIF D'ASSISTANCE CARDIAQUE POUR DÉTERMINER UNE PRESSION DE SORTIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.07.2014 EP 14175698**

(43) Veröffentlichungstag der Anmeldung:
**23.03.2022 Patentblatt 2022/12**

(62) Dokumentnummer(n) der früheren Anmeldung(en) nach Art. 76 EPÜ:
**15731980.7 / 3 164 168**

(73) Patentinhaber: **Berlin Heart GmbH**
**12247 Berlin (DE)**

(72) Erfinder: **KARCH, Dominik**
**12157 Berlin (DE)**

(74) Vertreter: **Pfenning, Meinig & Partner mbB**
**Patent- und Rechtsanwälte**
**Joachimsthaler Straße 10-12**
**10719 Berlin (DE)**

(56) Entgegenhaltungen:
**EP-A1- 2 298 375      US-A1- 2003 045 772**
**US-A1- 2004 039 243      US-A1- 2006 229 488**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Gegenstand dieser Anmeldung ist ein Verfahren zur Bestimmung eines an einem Auslass eines Herzunterstützungssystems anliegenden Auslassdrucks gemäß Anspruch 1 sowie ein Herzunterstützungssystem mit einer Pumpe und einer mit der Pumpe verbindbaren Kanüle gemäß Anspruch 9.

Bei den in dieser Anmeldung aufgeführten Herzunterstützungssystemen handelt es sich um sogenannte Ventricular Assist Devices (VAD). Diese können sowohl für den linken Ventrikel (LVAD), den rechten Ventrikel (RVAD) oder beide Ventrikel (BVAD) ausgebildet sein. Nachfolgend werden die Gegenstände dieser Anmeldung vorwiegend im Kontext von LVADs beschrieben, obgleich die jeweiligen Pumpen auch als RVADs oder BVADs einsetzbar sind.

Beim Betrieb eines Herzunterstützungssystems als LVAD wird das Herzunterstützungssystem zumeist mit dem linken Ventrikel und der Aorta, unter Umgehung der Aortenklappe, verbunden. Es wird also ein Bypass gelegt, welcher Blut durch das Herzunterstützungssystem vom linken Ventrikel in die Aorta pumpen kann. In zahlreichen Fällen umfasst das Herzunterstützungssystem eine Pumpeneinheit sowie eine Ein- bzw. Auslasskanüle. Andere Varianten sehen vor, die Pumpeneinheit direkt an das Herz zu koppeln und das durch die Pumpeneinheit geförderte Blut vom Pumpenauslass mittels einer Kanüle zur Aorta zu pumpen. Geeignete Pumpen sind hierbei rotierende Blutpumpen, welche entweder als Axialpumpen oder Radialpumpen ausgebildet sein können. Bei den Axialpumpen handelt es sich um Pumpen, welche das Blut axial fördern, d.h. das Blut durch eine vorwiegend axiale Vortriebskraft fördern. Die Pumpen zur axialen Blutförderung können mit einem radialen Auslass verbunden sein. Im Gegensatz zu den Axialpumpen besitzen Radialpumpen Rotoren, welche dem Blut neben einer Axialkomponente auch eine Radialkomponente aufzwingen. In vielen Fällen weitet sich der Rotor radial auf, so dass der Rotordurchmesser am Pumpeneinlass meist geringer ist als der Rotordurchmesser in der Nähe des Pumpenauslasses. Radialpumpen werden manchmal auch als Turbopumpen bezeichnet.

Um einen für den Patienten sicheren Betrieb des Herzunterstützungssystems zu gewährleisten, werden kontinuierlich Daten erfasst, um die geförderte Blutmenge und herrschende Drücke zu messen.

US 2003/0045772 A1 beschreibt eine Herzpumpe wobei basierend aus dem in einer Einlasskanüle gemessenen Einlassdrucks die Geschwindigkeit der Pumpe angepasst wird.

Eine Möglichkeit, die für die Herzunterstützungssysteme relevanten Drücke zu messen, besteht darin, Absolut-Drucksensoren im Herzunterstützungssystem vorzusehen. Relevante Drücke sind hier in einem LVAD-System unter anderem der Ventrikeldruck, $p_v$, und der Aortendruck, $p_{ao}$. Um den Ventrikeldruck zu messen, kann es vorgesehen sein, den Ventrikeldruck direkt im Ventrikel zu messen. Bei Herzunterstützungssystemen, bei welchen die Pumpeneinheit direkt an den Ventrikel angeschlossen wird, kann ein am Pumpeneinlass gemessener Druck im Wesentlichen mit dem Ventrikeldruck gleichgesetzt werden. Schwieriger gestaltet sich die Messung des Aortendrucks. In einer Variante kann ein Drucksensor beispielsweise in der Aorta angeordnet werden. Aufgrund der Drücke, der Flussgeschwindigkeiten und der schwierigen Zugänglichkeit der Aorta ist die Verankerung eines Sensors jedoch schwierig und birgt ein nicht zu vernachlässigendes Gesundheitsrisiko.

Von daher besteht Bedarf, den Aortendruck bzw. den Druck am Ausgang eines Herzunterstützungssystems auf andere Weise zu bestimmen. Neben den Verfahren nach Anspruch 1 s und einem Herzunterstützungssystem nach Anspruch 9 wird die Aufgabe auch durch die Anordnung eines Absolutdrucksensors am Auslass der Kanüle gelöst.

Gemäß einem Verfahren zur Bestimmung eines an einem Auslass eines Herzunterstützungssystems anliegenden Auslassdrucks wird zunächst ein an einem Einlass des Herzunterstützungssystems anliegender Einlassdruck bestimmt. Die Bestimmung des Einlassdrucks kann beispielsweise anhand eines Drucksensors, welcher am Einlass des Herzunterstützungssystems angeordnet ist, erfasst werden. Alternativ kann ein vom Herzunterstützungssystem separater Drucksensor verwendet werden, wie er beispielsweise in Herzschrittmachersystemen oder implantierbaren Defibrillatorsystemen zum Einsatz kommt. Die erfassten Druckdaten können dann beispielsweise drahtlos an eine Steuereinheit des Herzunterstützungssystems weitergeleitet werden.

Um den Auslassdruck zu bestimmen, wird eine zwischen dem Einlass und dem Auslass des Herzunterstützungssystems herrschende Druckdifferenz geschätzt. Dies bedeutet insbesondere, dass Teilabschnitte des Herzunterstützungssystems gemessen oder geschätzt werden können. Die Schätzung einer Druckdifferenz zwischen dem Einlass und dem Auslass des Herzunterstützungssystems kann also so verstanden werden, dass zumindest ein Druckabfall über einen Teilabschnitt des Herzunterstützungssystems geschätzt wird. Der Begriff "Schätzung" ist hierbei derart zu verstehen, dass der Druckabfall bzw. die Druckdifferenz mithilfe von Größen, welche nicht direkt in diesem Abschnitt gemessen werden, ermittelt wird.

In einer Ausführungsform umfasst das Herzunterstützungssystem eine Pumpe mit einem Pumpeneinlass und einem Pumpenauslass sowie eine Kanüle mit einem Kanüleneinlass und einem Kanülenauslass, wobei der Pumpenauslass mit dem Kanüleneinlass verbunden ist. Die Druckdifferenz zwischen dem Einlass und dem Auslass des Herzunterstützungssystems setzt sich hierbei aus einer Pumpendruckdifferenz zwischen dem Pumpeneinlass und dem Pumpenauslass und einer Kanülendruckdifferenz zwischen dem Kanüleneinlass und dem Kanülenauslass zusammen. Die Kanüle verbindet die Pumpe mit der Aorta und kann beispielsweise ein biokompatibles Material, wie z.B. Silikon oder ein Graftmaterial, aufweisen oder aus diesem Material gebildet sein. Da die Kanülen oftmals bei der Implantation individuell an den Patienten angepasst werden müssen, d.h., die Kanülen werden während der Implantation möglicherweise gekürzt, ist die abschließende Länge der Kanüle oftmals erst nach der Implantation bekannt. Mit Hilfe der in dieser Anmeldung beschriebenen Verfahren und des Herzunterstützungssystems kann die Kanüldruckdifferenz mit Hilfe von mittels der Pumpe gemessenen Parametern bzw. Größen geschätzt werden. Ist die Druckdifferenz zwischen dem Einlass und dem Auslass des Herzunterstützungssystems geschätzt, kann anschließend der Auslassdruck aus dem Einlassdruck und der geschätzten Druckdifferenz bestimmt werden.

Alternativ kann in einem Beispiel, welches nicht Teil der beanspruchten Erfindung ist, vorgesehen sein, am Auslass des mit der Aorta verbundenen Herzunterstützungssystems einen Drucksensor zur Ermittlung des Aortendrucks anzubringen. Ist ein Drucksensor nun am Auslass der Kanüle angeordnet, würde er gegebenenfalls entfernt werden. Alternativ, in einem Beispiel, welches nicht Teil der beanspruchten Erfindung ist, kann ein Drucksensor innerhalb der Kanüle vorgesehen sein, allerdings kann der in der Kanüle gemessene Druck vom Aortendruck abweichen, so dass in diesem Falle wiederum ein Druckabfall des Kanülenabschnitts zwischen dem Drucksensor und dem Kanülenauslass geschätzt werden sollte.

Ein Herzunterstützungssystem gemäß dieser Anmeldung umfasst eine Pumpe und eine mit der Pumpe verbindbare Kanüle. Ferner umfasst das Herzunterstützungssystem ein Druckerfassungssystem, welches derart konfigurierbar ist, dass ein am Pumpeneinlass anliegender Druck erfassbar ist und dass eine Pumpendruckdifferenz zwischen Pumpeneinlass und Pumpenauslass erfassbar ist und eine Steuerungseinheit vorhanden ist, welche derart konfiguriert ist, dass eine Kanülendruckdifferenz zwischen dem Kanüleneinlass und dem Kanülenauslass schätzbar ist. Wie bereits erwähnt, kann die Schätzung der Kanülendruckdifferenz anhand von Größen durchgeführt werden, welche mit der Pumpe, deren Druckerfassungssystem oder weiterer Sensorik der Pumpe erfassbar sind.

Hierbei ist die Steuerungseinheit, welche beispielsweise als Mikroprozessor, Mikrocontroller oder programmierbares Feldarray ausgebildet sein kann, mit einem Befehlssatz bzw. einer Software mit Instruktionen ausgestattet, welche mittels der von der Pumpe erfassten Messwerte und eines Modells zur Abschätzung der Kanülendruckdifferenz einen Schätzwert der Kanülendruckdifferenz ausgibt. Die Befehle bzw. die Soft- oder Firmware kann hierbei in einer Speichereinheit vorgehalten sein und/oder kann mittels eines Datenträgers, wie einer CD, einer DVD, eines transportierbaren Flashspeichers, vorgehalten und auf eine Steuereinheit übertragen werden. Die Steuereinheit ist derart konfiguriert, dass anhand des Modells zur Kanülendruckdifferenz und der zu dem Modell gehörigen, kalibrierten Modellparameter die Kanülendruckdifferenz aus mittels der Pumpe bestimmten Größen berechnet wird. Weiterhin umfasst die Steuereinheit in manchen Ausführungsbeispielen Befehle zur Durchführung eines Verfahrens zum Kalibrieren der Modellparameter.

In einer Ausführungsform kann das Modell zur Schätzung der Kanülendruckdifferenz als unabhängige Größen eine oder mehrere der nachfolgenden durch die Pumpe erfassbaren Größen beinhalten: Volumenstrom, Drehzahl der Pumpe oder die Pumpendruckdifferenz zwischen Pumpeneinlass und Pumpenauslass. Als abhängige Größe wird die Kanülendruckdifferenz oder der Kanülenauslassdruck geschätzt.

Um ein Modell zur Schätzung der Kanülendruckdifferenz an einen Patienten anzupassen, kann vorgesehen sein, Parameter des Modells anhand von Messdaten zu kalibrieren. Die Modellparameter können dabei beispielsweise mittels statistischer Methoden, wie beispielsweise einer Regressionsanalyse, modelliert werden. Die Modellierung der Parameter kann dabei im fortlaufenden Betrieb der Pumpe durchgeführt werden, die Parameter können in regelmäßigen Wartungsintervallen kalibriert werden, oder die Modellparameter können einmalig, beispielsweise kurz nach der Implantation, kalibriert werden. Das Kalibrieren der Modellparameter kann dabei entweder durch die Steuereinheit oder durch eine weitere, externe Steuereinheit durchgeführt werden. Die Modellparameter können zeitunabhängig oder zeitabhängig modelliert werden. Für den Fall, dass die Kalibrierung einmalig oder in regelmäßigen Abständen wiederholt wird, werden die anhand von statistischen Methoden bestimmten Modellparameter in einem mit der Steuereinheit verbundenen Speicher, wie beispielsweise einem RAM oder ROM, abgelegt und zur Bestimmung des Kanülendifferenzdrucks herangezogen.

Wie bereits eingangs erwähnt, ist die Kanülendruckdifferenz anhand verschiedener von der Pumpe erfassbarer Größen schätzbar. So kann beispielsweise in einer ersten Variante vorgesehen sein, die Kanülendruckdifferenz für Zeitpunkte zu schätzen, an welchen die Aortenklappe geöffnet ist, insbesondere zu Zeitpunkten kurz nach der Aortenklappenöffnung und vor dem Schließen der Aortenklappe. Zu diesen Zeitpunkten ist die Kanülendruckdifferenz in einer Näherung gleich der Pumpendruckdifferenz. Für diese erste Variante muss kein weiterer Parameter außer dem Zeitpunkt der Aortenklappenöffnung bestimmt werden. Nachteilig an dieser Methode ist jedoch, dass die Schätzung nur zu wenigen Zeitpunkten (kurz nach der Aortenklappenöffnung) zufriedenstellend genau ist.

In einer zweiten Variante kann vorgesehen sein, die Kanülendruckdifferenz mit Hilfe des durch die Pumpe geförderten Volumenstroms zu schätzen. So kann beispielsweise die Kanülendruckdifferenz über einen physiologisch sinnvollen Bereich durch ein Modell einer nichtlinearen Funktion, wie beispielsweise ein Polynom oder eine Exponentialfunktion, oder einer linearen Funktion geschätzt werden. Als unabhängige Größe können hierbei der Volumenstrom, die Drehzahl der Pumpe, die Pumpendruckdifferenz oder Kombinationen dieser Größen oder zu diesen Größen proportionale Größen dienen. So kann beispielsweise ein Polynom oder eine Exponentialfunktion des Pumpendifferenzdrucks und der Drehzahl des Rotors zur Schätzung der Kanülendruckdifferenz herangezogen werden. Weiterhin ist es beispielsweise bei manchen Pumpen im Stand der Technik möglich, die Pumpendruckdifferenz anhand eines an einer Steuerspule anliegenden Stroms zu bestimmen, wobei die Steuerspule eine Position eines Pumpenrotors bestimmt. Da die Position aufgrund des anliegenden Drucks bzw. des herrschenden Volumenstroms durch das geförderte Fluid verändert wird, ist der durch die Steuerspule fließende Strom proportional zu einer Pumpendruckdifferenz.

In einer dritten Variante kann das Modell der Kanülendruckdifferenz eine Differentialgleichung oder ein Differentialgleichungssystem aufweisen.

Der zweiten und dritten Variante ist hierbei gemein, dass das Modell Modellparameter umfasst, welche beispielsweise anhand statistischer Methoden aus Messdaten ermittelt werden. Beispielhafte Verfahren zum Kalibrieren der Modelle finden sich in den Ausführungsbeispielen.

In einer weiteren Ausführungsform des Herzunterstützungssystems umfasst dieses einen Volumenstromsensor. Mittels des Volumenstromsensors kann der Volumenstrom direkt ermittelt werden. Als Volumenstromsensor kann beispielsweise ein Ultraschalldurchflusssensor, eine Prandtlsonde, eine Messblende oder ein magnetisch induktiver Durchflusssensor verwendet werden. Ein Pumpensystem zur indirekten Messung des Volumenstroms, beispielsweise mittels einer Verschiebung eines Rotors, fällt nicht unter den Begriff des Volumenstromsensors.

In einer weiteren Ausführungsform umfasst das Druckerfassungssystem einen Drucksensor am Pumpeneinlass und einen Drucksensor am Pumpenauslass. Anhand dieser Absolutdrucksensoren kann der Pumpendifferenzdruck bestimmt werden. Ein am Pumpeneinlass angeordneter Drucksensor ist beispielsweise geeignet, den Ventrikeldruck eines Herzunterstützungssystems zu bestimmen. Alternativ oder in Ergänzung zu einem Sensor am Pumpenein- und/oder -auslass kann ein Pumpensystem zur Ermittlung eines Differenzdrucks vorhanden sein. Ein derartiges System kann beispielsweise mittels eines Systems zum Erfassen und zum Ausgleichen eines Pumpenrotorversatzes gebildet sein. Hierzu bedarf es beispielsweise eines Kennfeldes, welches den entsprechenden durch mindestens eine Steuerspule des Systems fließenden Strom zum Verschieben des Pumpenrotors mit dem entsprechenden Differenzdruck bzw. Volumenstrom in Verbindung bringt.

Weitere Ausführungsbeispiele finden sich in den nachstehend aufgeführten Beispielen.

Es zeigen:

Fig. 1 einen schematischen Aufbau eines Herzunterstützungssystems;

Fig. 2a einen beispielhaften Aufbau einer Pumpe mit daran angebundener Kanüle;

Fig. 2b einen schematischen Aufbau einer Steuerungseinheit des Herzunterstützungssystems,

Fig. 3 ein schematisches Ablaufdiagramm für ein Verfahren zur Bestimmung eines an einem Auslass eines Herzunterstützungssystems anliegenden Auslassdrucks;

Fig. 4 eine schematische Darstellung der Schätzung der Pumpendruckdifferenz eines Herzunterstützungssystems mit einer Pumpe und einer Kanüle; und

Fig. 5 eine schematische Übersicht über ein Verfahren zur Bestimmung von Modellparametern für ein Modell zur Bestimmung der Kanülendruckdifferenz.

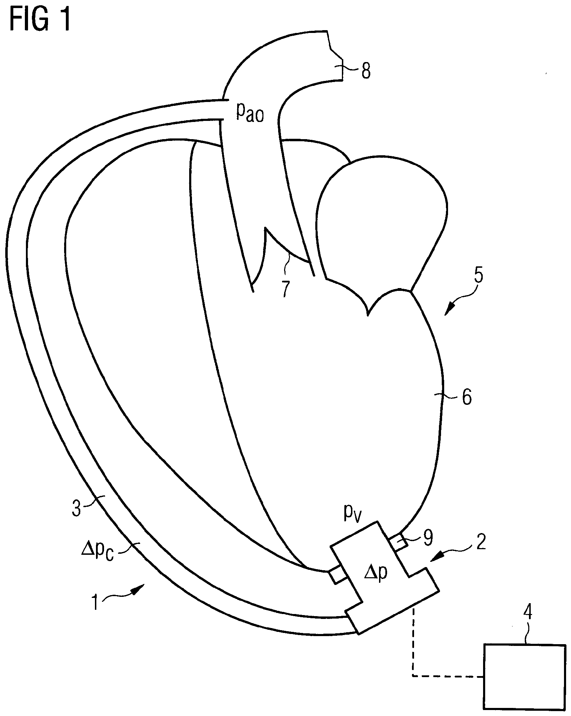

Figur 1 zeigt den schematischen Aufbau eines implantierten Herzunterstützungssystems 1. Das beispielhafte Herzunterstützungssystem 1 umfasst eine Pumpe 2, welche an ihrem Auslass mit einer Kanüle 3 verbunden ist. Bei der Pumpeneinheit bzw. Pumpe 2 kann es sich sowohl um eine Axialpumpe als auch um eine Radialpumpe handeln. Die Pumpen können dabei beispielsweise mittels der Steuereinheit 4 derart eingestellt werden, dass sie eine vorgegebene Menge an Blut fördern. Allerdings sind auch andere Förderprogramme möglich, wie beispielsweise eine Förderungsart, welche den Schlag eines natürlichen Herzens genauer abbildet.

Die Kanüle 3 kann beispielsweise aus Silikon oder einem weicheren Graftmaterial, wie beispielsweise einem Polyester, ePTFE oder Mischungen dieser Materialien, geformt sein. Die Steuereinheit 4 kann entweder vollständig an der Pumpe 2 befestigt, teilweise an der Pumpe 2 befestigt oder vollständig von der Pumpeneinheit 2 entfernt angeordnet sein. Die Steuereinheit 4 kann elektronische bzw. elektrische Steuerelemente, wie beispielsweise einen Prozessor, Speicher, Signalverarbeitungsschaltkreise, Motorsteuerungsschaltkreise sowie Überwachungssysteme, umfassen.

Bei der dargestellten Pumpe 2 handelt es sich um eine Axialpumpe, welche mit ihrem Einlass direkt an ein Herz 5 gekoppelt ist. In dem vorliegenden Beispiel handelt es sich bei dem Herzunterstützungssystem um ein LVAD-System. Physiologisch ist der linke Ventrikel 6 über die Aortenklappe 7 mit der Aorta 8 verbunden. Das Herzunterstützungssystem 1 stellt einen Bypass vom linken Ventrikel 6 zur Aorta 8 dar. Bei einem RVAD-System wäre die Pumpe mit dem rechten Ventrikel und der Pulmonararterie verbunden, bei einem BVAD-System wäre eine Doppelkammerpumpe vorgesehen, welche mit der ersten Kammer ein LVAD-System und mit der zweiten Kammer ein RVAD-System bildet. Alternativ können zwei Pumpen vorhanden sein.

Im vorliegenden Beispiel eines LVAD-Systems wird Blut vom linken Ventrikel 6 durch die Pumpeneinheit 2 und die Kanüle 3 gefördert, wobei der Auslass der Kanüle 3 mit der Aorta 8 verbunden ist. In Figur 1 ist weiterhin ein Nahtring 9 dargestellt, mit welchem die Pumpe 2 am Apex des Herzens befestigt ist.

Im linken Ventrikel 6 herrscht ein Ventrikeldruck $p_v$, und in der Aorta herrscht ein Aortendruck $p_{ao}$. Physiologisch gesehen öffnet die Aortenklappe 7, wenn der Druck $p_v$ im Ventrikel größer ist als der Aortendruck $p_{ao}$. Der Aortendruck $p_{ao}$ lässt sich als Summe des Ventrikeldrucks $p_v$, des Druckabfalls $\Delta p$ über der Pumpe 2 sowie des Druckabfalls $\Delta p_c$ über der Kanüle 3 beschreiben, d.h.

$$p_{ao} = p_v + \Delta p + \Delta p_c.$$

Eine Möglichkeit zur Ermittlung des Aortendrucks bestünde beispielsweise darin, einen zusätzlichen Drucksensor in der Aorta 8 zu platzieren. Die Implantation ist jedoch mit einem gewissen gesundheitlichen Risiko verbunden. Alternativ hierzu kann am oder im Auslass der Kanüle 3, welcher mit der Aorta 8 verbunden ist, ein Drucksensor zur Erfassung des Drucks am Auslass der Kanüle angeordnet werden. Obgleich diese Variante die direkte Messung des Aortendrucks zulässt, erfordert der in der Kanüle angeordnete Sensor eine datentechnische Anbindung an die Steuereinheit 4. Da die Kanüle 3 bei der Implantation häufig gekürzt werden muss bzw. wird, um erst anschließend mit der Aorta 8 verbunden zu werden, kann nicht sichergestellt werden, dass ein am Auslass der Kanüle 3 angeordneter Drucksensor nach dem Zuschneiden der Kanüle noch vorhanden ist. Eine mögliche Lösung ist hierbei das Versetzen des Drucksensors in die Kanüle 3 hinein. In diesem Fall jedoch muss zumindest der Druckabfall zwischen dem Ort des Sensors und dem mit der Aorta 8 verbundenen Auslass der Kanüle 3 geschätzt werden.

Dem in dieser Anmeldung vorgestellten Verfahren liegt eine Möglichkeit zugrunde, den Aortendruck $p_{ao}$ zu bestimmen, beispielsweise indem der Druckabfall $\Delta p_c$ geschätzt wird. Sind die weiteren Größen messtechnisch erfassbar, d.h. $p_v$ und $\Delta p$, kann aus den gemessenen Größen des Ventrikeldrucks und des Druckabfalls über die Pumpe der Aortendruck bestimmt werden. Obgleich in Figur 1 das Herzunterstützungssystem 1 lediglich eine Auslasskanüle umfasst, kann das Herzunterstützungssystem auch eine Einlasskanüle umfassen. Die hier vorgestellten Verfahren können auch bei einem derartigen Herzunterstützungssystem zur Anwendung kommen.

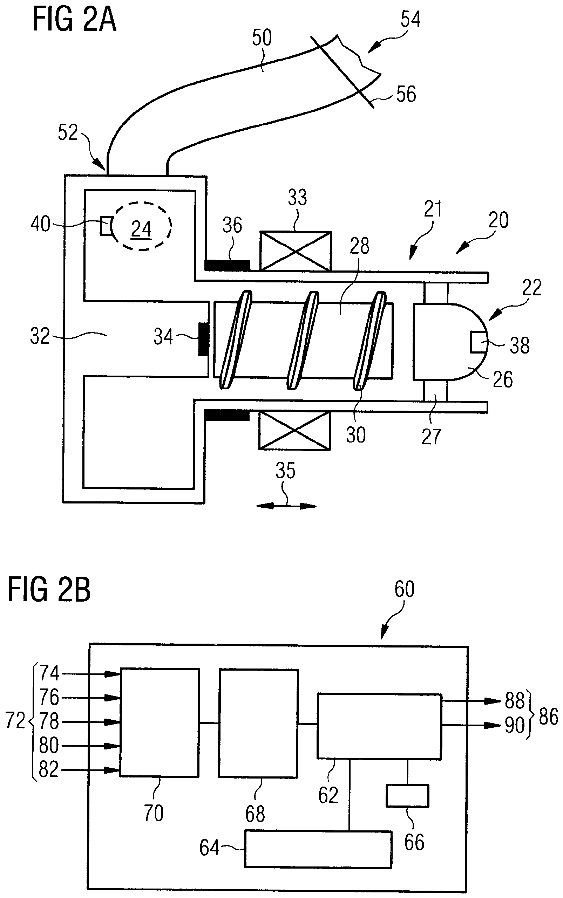

In Figur 2a wird ein beispielhaftes Herzunterstützungssystem 20 näher gezeigt. Das Herzunterstützungssystem umfasst eine Pumpe 21 mit einem Pumpeneinlass 22 und einem Pumpenauslass 24. Die Pumpeneinheit pumpt Blut vom Pumpeneinlass 22 an einem Vorleitrad 26 vorbei, welches über Flügel 27 mit einer Gehäusewand der Pumpe 21 verbunden ist, zu einem beispielsweise als Rotor ausgebildeten Förderelement 28. Das Förderelement 28 besitzt im vorliegenden Beispiel eine Wendel 30, welche das Blut auf das Nachleitelement 32 zu fördert. Das Förderelement 28 wird dabei mittels eines Motors 33 in Rotation versetzt. Hierzu befinden sich in dem Förderelement 28 Permanentmagnete, welche durch den Motorstrom des Motors 33 in Rotation versetzt werden können. Das Nachleitrad bzw. das Nachleitelement 32 ist in einer Spiralkammer angeordnet, in welche das axial geförderte Blut gefördert und anschließend durch den Pumpenauslass 24 hinausgedrückt wird.

Beispiele für derartige Pumpen können unter anderem den Druckschriften WO 2013/021014, WO 2012/150045, WO 2012/149946, WO 2011/054545 und WO 02/066837 entnommen werden. Weitere exemplarische Pumpen sind beispielsweise in der WO 2012/051454 oder WO 2012/024493 gezeigt.

Im vorliegenden Ausführungsbeispiel umfasst die Pumpe 21 ein Druckerfassungssystem. Dieses umfasst beispielsweise eine Sensorspule 34, welche einen Abstand des Förderelements 28 zum Nachleitkörper 32 erfassen kann. Wirkt aufgrund des geförderten Blutstroms eine entlang den Richtungen 35 gerichtete Kraft auf das Förderelement 28, wird dieses aus einer Ruhelage ausgelenkt und hierdurch ein Strom in der Sensorspule 34 induziert. Dieser induzierte Strom wird erfasst und in der Steuereinheit oder einem anderen Schaltkreis derart verarbeitet, dass durch Anlegen eines Stroms in einer Steuerspule 36 das Förderelement 28 wieder in eine zur Ruheposition korrespondierende Position zwischen dem Vorleitrad 26 und dem Nachleitkörper 32 gebracht wird. Anhand des an der Steuerspule 36 anliegenden Stroms (direkt oder indirekt gemessen) kann beispielsweise eine Druckdifferenz zwischen dem Pumpeneinlass 22 und dem Pumpenauslass 24 ermittelt werden.

Weiterhin kann das Druckerfassungssystem einen Drucksensor 38 umfassen, welcher am Einlass 22 der Pumpe 31 angeordnet ist. Dieser Drucksensor erfasst den Blutdruck am Einlass der Pumpe, so dass, wie in Figur 1 gezeigt, beispielsweise ein Ventrikeldruck $p_v$ bestimmbar ist. Das Druckerfassungssystem kann ferner einen weiteren Drucksensor 40 umfassen, welcher den Druck am Auslass 24 der Pumpe erfasst. Umfasst die Pumpe zwei Sensoren am Einlass und Auslass, kann die Pumpendruckdifferenz aus den beiden gemessenen Werten ermittelt werden. Zumindest einer der beiden Drucksensoren kann jedoch auch durch ein Differenzdrucksensorsystem, wie beispielsweise das die Elemente 34 und 36 umfassende System zum indirekten Erfassen des Differenzdrucks der Pumpe, ersetzt bzw. ergänzt werden. Die verschiedenen mithilfe des Druckerfassungssystems erfassten Daten werden durch eine Steuereinheit erfasst, verarbeitet und zur Schätzung des Aortendrucks verwendet.

Das Herzunterstützungssystem 20 umfasst ferner eine Kanüle 50, welche beispielsweise aus einem Silikon gefertigt ist. Ein Kanüleneinlass 52 ist dabei mit dem Pumpenauslass 24 beispielsweise über einen Snap-Fit-Connector verbunden. Am Kanülenauslass 54 kann die Kanüle 50 mit einer Aorta verbunden, beispielsweise vernäht werden. Vor dem Verbinden der Aorta mit der Kanüle kann es notwendig sein, die Kanüle beispielsweise auf eine Länge 56 zu kürzen. Daher ist es in einigen Ausführungsbeispielen vorgesehen, keine aktive Sensorik in der Kanüle bereitzustellen. Allerdings kann das hier vorgestellte System bzw. das hier vorgestellte Verfahren auch dazu verwendet werden, eine aktive Sensorik in der Kanüle zu komplementieren.

Sind in der Kanüle keine Sensorkomponenten vorhanden, wird mithilfe eines in dieser Anmeldung erläuterten Verfahrens der Druckabfall zwischen dem Kanüleneinlass 52 und dem Kanülenauslass 54 bzw. dem gekürzten Kanülenauslass 56 geschätzt. Schätzen bedeutet hierbei, dass beim Bestimmen des Druckabfalls $\Delta p_c$ entlang der Kanüle 50 lediglich auf mittels der Pumpe 21 bestimmte Größen oder anhand von außerhalb der Pumpe oder Kanüle gemessene Größen zurückgegriffen werden kann. Hierzu sind verschiedene Modelle zur Kanülendruckabfall- bzw. Kanülendruckdifferenzschätzung möglich.

In Figur 2b ist eine exemplarische Steuereinheit 60 schematisch dargestellt. Die Steuereinheit 60 kann beispielsweise in einem externen Gehäuse außerhalb des Körpers angeordnet sein. Von der Pumpeneinheit erfasste Daten werden beispielsweise über einen Datenbus an die Steuereinheit 60 weitergegeben. Allerdings kann die Steuereinheit auch an der Pumpeneinheit befestigt, beispielsweise in ein Pumpengehäuse integriert sein. Die hier dargestellte Steuereinheit umfasst eine CPU 62, welche zum Betrieb des Herzunterstützungssystems konfiguriert ist. Das heißt, sie ist insbesondere in der Lage, die Drehzahl des Herzunterstützungssystems einzustellen.

Die CPU 62 ist mit einer ersten Speichereinheit 64 verbunden, in welcher verschiedene Befehle und Steuerungsprogramme abgelegt sind. Im vorliegenden Beispiel umfassen die Steuerbefehle auch Instruktionen zum Ermitteln eines geschätzten Aortendrucks und Instruktionen zum Ermitteln von Modellparametern eines Modells zur Aortendruckschätzung bzw. Kanülendruckdifferenzschätzung. Die CPU 62 kann weiterhin mit einem weiteren Speicher 66 verbunden sein, welcher beispielsweise zur Aufzeichnung von Daten und zum späteren Auslesen der Daten durch einen Benutzer konfiguriert ist.

Der CPU vorgeschaltet ist eine Signalverarbeitungsstufe 68, welche eingehende Signale, die am Dateneingang 70 eintreffen, filtert, verarbeitet und aufbereitet, so dass die Daten durch die CPU 62 verarbeitbar sind. Neben Filtern kann die Signalverarbeitungsstufe 68 beispielsweise Analog/Digital-Wandler oder Ähnliches umfassen.

Am Dateneingang 70 laufen Signale 72 ein. Beispielhaft umfassen diese Signale gemessene Motorströme 74 zur Ermittlung der Drehzahl, Signale des Absolutdrucksensors 38 in Form der Signale 76 und weiterhin Signale 78, welche Signale des Absolutdrucksensors 40 repräsentieren. Signale 80 repräsentieren den in der Steuerspule 36 anliegenden Strom, welcher durch die CPU 62 in einen Pumpendifferenzdruck gewandelt werden kann. Optional kann das in Figur 2a dargestellte Pumpensystem auch einen Volumenstromsensor umfassen, dessen Signale 82 ebenfalls am Dateneingang 70 eingehen. Die CPU 62 kann weiterhin Signale an die Pumpe zurückleiten, wie beispielsweise die Signale 86, welche beispielhaft als Ansteuerungsströme 88 für den Motor oder Ströme für die Steuerungsspule 36 dargestellt sind.

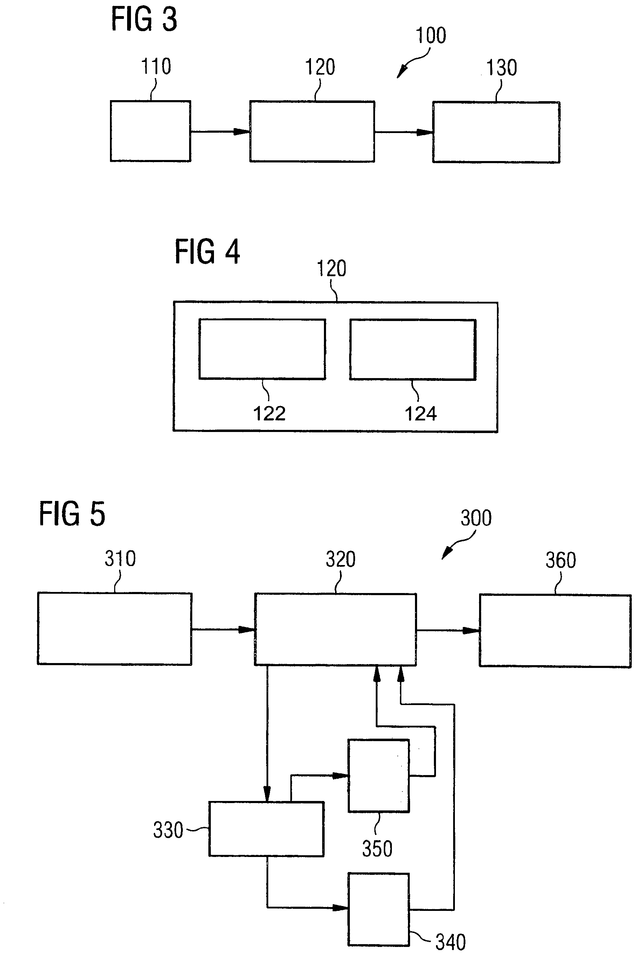

Anhand der Figur 3 soll ein Verfahren zur Bestimmung des Aortendrucks (auch Nachlast des Herzunterstützungssystems genannt) erläutert werden. Das Verfahren 100 zur Bestimmung des Aorten- bzw. Auslassdrucks des Herzunterstützungssystems beinhaltet den Schritt 110, in welchem zunächst ein Druck im Ventrikel bzw. am Herzunterstützungssystemeinlass gemessen wird. Diese Messung kann beispielsweise mittels eines Absolutdrucksensors am Einlass des Herzunterstützungssystems vorgenommen werden. Anschließend wird mithilfe eines kalibrierten Modells ein Differenzdruck zwischen dem Einlass und dem Auslass des Herzunterstützungssystems geschätzt. Dies erfolgt in Schritt 120. Aus der geschätzten Druckdifferenz und dem gemessenen Einlassdruck kann anschließend der Aortendruck bzw. Auslassdruck im Schritt 130 ermittelt werden.

Der Schritt 120 wird erfindungsgemäß bei einem Herzunterstützungssystem mit einer Pumpe und einer daran angeschlossenen Kanüle wie in Figur 4 illustriert derart in Einzelschritte unterteilt , dass zunächst eine Pumpendruckdifferenz im Schritt 122 gemessen wird und anschließend eine Kanülendruckdifferenz anhand der mithilfe der Pumpe gemessenen Größen und eines Modells der Kanülendruckdifferenz geschätzt wird (Schritt 124).

Anhand der Figur 5 sollen verschiedene Modelle der Kanülendruckdifferenz erläutert werden, Möglichkeiten zur Kalibrierung bzw. Bestimmung der etwaigen Modellparameter gegeben werden und erläutert werden, wie das mit den Modellparametern kalibrierte Modell in dem Herzunterstützungssystem zur Anwendung kommt.

Das beispielhafte Verfahren 300 umfasst zunächst die Bestimmung eines Modells der Kanülendruckdifferenz 310. Hier können verschiedene Modelle in Erwägung gezogen werden. Einige Modelle seien hier exemplarisch aufgeführt.

In einem ersten Modell kann die Kanülendruckdifferenz $\Delta p_c$ zu ausgewählten Zeitpunkten durch die Pumpendruckdifferenz $\Delta p$ modelliert werden. So ist beispielsweise bekannt, dass zum Zeitpunkt der Aortenklappenöffnung der Ventrikeldruck $p_v$ kurzzeitig dem Aortendruck $p_{ao}$ im Wesentlichen gleich ist. Für diese Zeitpunkte kann also das Modell aufgestellt werden, dass die Kanülendruckdifferenz gleich der Pumpendruckdifferenz ist. Allerdings ist dieses Modell nur zu wenigen Zeitpunkten während eines einzelnen Herzzyklus anwendbar, da die Abweichungen zwischen dem Aorten- und dem Ventrikeldruck zu anderen Zeitpunkten als zum Zeitpunkt der Aortenklappenöffnung sich deutlich voneinander unterscheiden. D.h., die Zeitpunkte, zu denen das Modell Gültigkeit besitzt, müssen fortlaufend bestimmt werden und bilden in diesem Sinne die Modellparameter. Hierfür ist ein Verfahren notwendig, welches zuverlässig die Öffnung und insbesondere den Zeitpunkt der Öffnung der Aortenklappe bestimmen kann. Anschließend muss noch ein Zeitintervall ab der Öffnung festgelegt werden, in welchem das Modell Anwendung findet. Ist die Pumpe bzw. deren Steuereinheit in der Lage, die Aortenklappenöffnung zu bestimmen, kann eine Steuereinheit eines Herzunterstützungssystems zumindest zu den Zeitpunkten der Herzklappenöffnung den Aortendruck bestimmen. Nachteil des Modells ist es jedoch, dass eine kontinuierliche Bestimmung des Aortendrucks keine zufriedenstellenden Ergebnisse liefert.

Ein weiteres Modell zur Bestimmung der Kanülendruckdifferenz ist eine Beziehung zwischen einem statischen Volumenstrom, welcher beispielsweise mittels durch die Pumpe ermittelbarer Größen bestimmbar ist. So kann die Druckdifferenz $\Delta p_c$ beispielsweise durch eine nichtlineare Funktion approximiert werden. Als ein Beispiel sei hier ein Polynom in dritter Ordnung des Volumenstroms angeführt. Alternativ können jedoch auch andere nichtlineare Funktionen, wie beispielsweise eine Exponentialfunktion, angewandt werden. Das Polynom kann beispielsweise wie folgt dargestellt werden:

$$\Delta p_c = k_3 \dot{Q}_p^3 + k_2 \dot{Q}_p^2 + k_1 \dot{Q}_p^1 \ ,$$

wobei $\dot{Q}$ den Volumenstrom durch die Pumpe darstellt und die Modellparameter $k_1$, $k_2$ und $k_3$ bestimmt werden müssen.

Um die Modellparameter $k_1$, $k_2$, $k_3$ zu bestimmen, ist es in einer Ausführungsform vorgesehen, dass der Druckabfall $\Delta p_c$ über der Kanüle zu bestimmten Messpunkten bekannt ist, der Volumenstrom näherungsweise konstant ist und mindestens drei verschiedene Messpunkte bekannt sind. Sind die Modellparameter jedoch bestimmt bzw. kalibriert, kann das Modell zur fortlaufenden Schätzung der Kanülendruckdifferenz angewandt werden, d.h., es liefert auch in Herzzyklenabschnitten mit geschlossener Aortenklappe zufriedenstellende Ergebnisse. Vorteil ist hierbei, dass die Kalibrierung des Modells nur einmal erfolgen muss, wobei eine Kalibrierung in bestimmten, beispielsweise regelmäßigen Zeitintervallen, wie Tagen, Wochen oder Monaten, durchgeführt werden kann.

Wie bereits im ersten Modell beschrieben, ist zumindest zu Zeitpunkten kurz nach der Öffnung der Aortenklappe der Druckabfall in der Kanüle $\Delta p_c$ im Wesentlichen gleich dem Druckabfall in der Pumpe. Daher kann wenigstens für diese Zeitpunkte die Kanülendruckdifferenz ermittelt werden. Zu diesen Zeitpunkten ist die Druckdifferenz zwischen dem Ventrikel und der Aorta näherungsweise konstant. Daher sind auch die Volumenströme durch die Aortenklappe und durch die Pumpe näherungsweise konstant. Da der Volumenstrom während der Austreibungsphase des Ventrikels zwar näherungsweise konstant, nicht jedoch absolut konstant ist, können Grenzwerte festgelegt werden, anhand deren bestimmt wird, dass der Volumenstromabfall während der Austreibungsphase des Herzens zufriedenstellend konstant ist. Hier kann beispielsweise die Standardabweichung des Volumenstroms während der Austreibungsphase ermittelt werden und ein Grenzwert festgelegt werden, unterhalb dessen das Austreibungsphasenintervall zur Bestimmung der Modellparameter anwendbar ist.

Um weiterhin die Parameter für einen großen Drehzahlbereich der Pumpe bestimmen zu können, werden die Messungen zu mehreren, beispielsweise drei unterschiedlichen Drehzahlen n durchgeführt und die zugehörigen Daten bzw. Zeitreihen aufgenommen. Für die während der jeweiligen Drehzahl aufgenommenen Zeitreihen der Pumpendruckdifferenz und des Volumenstroms durch die Pumpe in Verbindung mit den Kriterien, dass der Volumenstrom zufriedenstellend konstant ist und die Kanülendruckdifferenz mit der Pumpendruckdifferenz im Wesentlichen gleichgesetzt werden kann, werden nun die Modellparameter $k_1$, $k_2$, $k_3$ bestimmt. Hierbei kann beispielsweise folgender Zusammenhang gelten:

$$\vec{k} = \Delta p(\vec{t}) \cdot \hat{\dot{Q}}^+(t)$$

Hierbei ist $\hat{\dot{Q}}^+$ die Pseudoinverse der Matrix $\hat{\dot{Q}}$, welche zu verschiedenen Messzeitpunkten aufgenommene Werte $\dot{Q}_p^3$, $\dot{Q}_p^2$ und $\dot{Q}_p^1$ als Einträge besitzt. Während der k-Vektor in diesem Ausführungsbeispiel dreidimensional ist, ist der Pumpendruckdifferenzvektor m-dimensional, sofern m Messpunkte vorliegen. Die Matrix $\hat{\dot{Q}}$ ist eine [m×3]-Matrix. Mittels statistischer Methoden, wie beispielsweise einer Regression, können also die Parameter $k_1$, $k_2$, $k_3$ bestimmt und validiert werden. Sobald die Modellparameter $k_1$, $k_2$, $k_3$ bestimmt worden sind, kann die Kanülendruckdifferenz mithilfe des durch die Pumpe fließenden Volumenstroms zu beliebigen Zeitpunkten des Herzzyklus durch das Modell geschätzt werden.

Somit werden im Schritt 330 zunächst die Zeitpunkte der Aortenklappenöffnung zu unterschiedlichen Drehzahlen bestimmt. Die entsprechenden Messdaten des Volumenstroms und der Pumpendruckdifferenz werden in den Schritten 340 und 350 erfasst. Danach wird aus den erfassten Daten unter den beschriebenen Randbedingungen eine Kalibrierung der Modellparameter, z.B. eine Bestimmung der Modellparameter in der gewünschten Genauigkeit, durchgeführt, so dass die Modellparameter im Schritt 320 kalibriert sind. Anschließend kann das Modell durch die Steuereinheit angewendet werden (Schritt 360), so dass durch Messung des Volumenstroms, der Pumpendruckdifferenz und des Pumpeneinlassdrucks eine Aussage über den Pumpen- bzw. Kanülenauslassdruck, welcher mit dem Aortendruck nahezu übereinstimmt, gemacht werden kann.

Ein weiteres Modell ist die Verwendung eines Differentialgleichungssystems zur Bestimmung der Kanülendruckdifferenz. Während das im vorhergehenden Abschnitt beschriebene Modell im Wesentlichen nach einer statischen Kanülendruckdifferenz modelliert ist, kann durch eine Differentialgleichung auch ein dynamischer Kanülendruckdifferenzterm erfasst werden. Eine Möglichkeit, die Kanülendruckdifferenz $\Delta p_c$ zu modellieren, ist beispielsweise

$$\Delta p_c = L_c \frac{d\dot{Q}_p}{dt} + R_c\left(\dot{Q}_p\right) \cdot \dot{Q}_p \quad ,$$

wobei $R_c\left(\dot{Q}_p\right)$. $\dot{Q}_p$ dem statischen Kanülendruckdifferenzterm der zweiten Variante entsprechen kann ($R_c$ entspricht einem Widerstandsterm in einem Ersatzschaltbild) und $L_c$ eine Massenträgheit des Fluidvolumens der Kanüle und der Pumpe modelliert. Die Massenträgheit des Fluidvolumens in der Pumpe lässt sich aufgrund der Geometrie der Pumpe bestimmen. Die Massenträgheit $L_c$* des Fluidvolumens der Kanüle lässt sich beispielsweise bei einer im Innenquerschnitt runden Ka-

$$L_c = \frac{4\rho l}{\pi D_i^2}$$

nüle der Länge l durch den Term                bestimmen, wobei $\rho$ die Dichte des Blutes, l die Länge der Kanüle und $D_i$ der Durchmesser der Kanüle ist. Andere Geometrien einer Kanüle können beispielsweise durch Anpassung des Massenträgheitsterms erfasst werden (z.B. durch Integration aller Teilabschnitte der Kanüle) und sind nach den obigen Ausführungen für den Fachmann ersichtlich. Die Massenträgheit kann für bekannte Kanülen auch experimentell als numerischer Wert bestimmt werden.

Bei der Verwendung dieses Modells kann ähnlich wie im vorhergehenden Fall vorgegangen werden, indem zuerst die statischen Parameter $k_1$ bis $k_3$ bestimmt werden. Anschließend werden beispielsweise die Länge der Kanüle und deren bekannter Innendurchmesser ermittelt und an die Steuereinheit übertragen. Anhand der gesammelten Informationen kann dann die Kanülendruckdifferenz geschätzt werden.

Als Erweiterung dieser Modelle ließe sich weiterhin der Druckabfall über die Aortenklappe integrieren. Dieser könnte z.B. auf Basis einer angenommenen oder aufgrund von bildgebenden Verfahren vermessenen Geometrie der Aortenklappe geschätzt werden.

Weiterhin wird in den bisher dargestellten Modellen der Einfluss der Elastizität der Kanülen ignoriert, da die Kanülen eine hohe Elastizität aufweisen und somit keine Wellenreflexionen im interessierenden Frequenzbereich zu erwarten sind. Es ist allerdings denkbar, einen Term für die Elastizität der Kanüle im Bereich der Aortenanastomose in die Modelle einzubeziehen; dies könnte eventuell die Genauigkeit der Schätzung verbessern.

Obgleich bei den hier vorgestellten Modellen zur Bestimmung der Kanülendruckdifferenz lediglich Modelle vorgestellt wurden, welche als unabhängige Größe den Volumenstrom beinhalten, können ähnliche Modelle auch für eine Pumpendruckdifferenz bzw. eine Drehzahl oder eine Kombination dieser Größen aufgestellt werden.

Ist ein Modell für die Kanülendruckdifferenz aufgestellt, kann der Auslass- bzw. Aortendruck aus dem Pumpeneinlass- bzw. Ventrikeldruck, dem Pumpendruckabfall und dem Modell bestimmt werden.

**Patentansprüche**

1. Verfahren zur Bestimmung eines an einem Auslass eines eine Pumpe und daran angeschlossenen Kanüle umfassenden Herzunterstützungssystems (1; 20) anliegenden Auslassdrucks ($p_{ao}$), insbesondere eines Aortendrucks, wobei das Verfahren folgende Schritte umfasst:

   - Bestimmung eines an einem Einlass des Herzunterstützungssystems anliegenden Einlassdrucks ($p_v$);
   - Erfassen einer Pumpendruckdifferenz ($\Delta p$) zwischen Pumpeneinlass und Pumpenauslass der Pumpe des Herzunterstützungssystems;
   - Schätzung einer Druckdifferenz zwischen dem Einlass und dem Auslass des Herzunterstützungssystems basierend auf der Pumpendruckdifferenz ($\Delta p$) und einer geschätzten Kanülendruckdifferenz ($\Delta p_c$);
   - Bestimmung des Auslassdrucks aus dem Einlassdruck und der Druckdifferenz.

2. Verfahren nach Anspruch 1, wobei der Einlassdruck anhand eines Drucksensors bestimmt wird.

3. Verfahren nach Anspruch 1 oder 2, wobei das Erfassen der Pumpendruckdifferenz anhand eines an einer Steuerspule anliegenden Stroms erfolgt, wobei die Steuerspule eine Position eines Pumpenrotors bestimmt.

**4.** Verfahren nach Anspruch 1 oder 2, wobei die Pumpendruckdifferenz durch zwei Drucksensoren die am Einlass und Auslass der Pumpe angeordnet sind oder durch einen in der Pumpe angeordneten Differenzdrucksensor erfasst wird.

**5.** Verfahren nach Anspruch 1, wobei die Druckdifferenz durch eine nichtlineare Funktion geschätzt wird, oder wobei die Druckdifferenz durch eine nichtlineare Funktion eines Volumenstroms approximiert wird, wobei Parameter der nichtlinearen Funktion aus Messpunkten, an denen die Druckdifferenz bekannt ist, bestimmt werden.

**6.** Verfahren nach einem der vorhergehenden Ansprüche, wobei ein dynamischer Druckdifferenzterm durch eine Differentialgleichung erfasst wird.

**7.** Verfahren nach einem der vorhergehenden Ansprüche, wobei ein Modell der Druckdifferenz bestimmt und mindestens ein Parameter des Modells anhand statistischer Methoden ermittelt wird.

**8.** Verfahren nach einem der vorhergehenden Ansprüche, wobei die Druckdifferenz mittels eines Polynoms höherer Ordnung geschätzt wird und wobei das Polynom vorzugsweise eine Funktion einer durch das Herzunterstützungssystem messbaren Größe ist.

**9.** Herzunterstützungssystem (1; 20) mit einer Pumpe (2; 21) zur Herzunterstützung und einer mit der Pumpe verbindbaren Kanüle (3; 50), wobei die Pumpe einen Pumpeneinlass (22) und einen Pumpenauslass (24) und die Kanüle einen Kanüleneinlass (52) und einen Kanülenauslass (54) umfasst und das Herzunterstützungssystem ein Druckerfassungssystem aufweist, welches derart konfigurierbar ist, dass ein am Pumpeneinlass anliegender Druck erfassbar ist und dass eine Pumpendruckdifferenz zwischen Pumpeneinlass und Pumpenauslass erfassbar ist, und wobei eine Steuerungseinheit (60) vorhanden ist, welche derart konfiguriert ist, dass eine Kanülendruckdifferenz ($\Delta p_c$) zwischen dem Kanüleneinlass und dem Kanülenauslass anhand der Pumpendruckdifferenz schätzbar ist.

**10.** Herzunterstützungssystem nach Anspruch 9, wobei die Steuereinheit eine Speichereinheit (64, 66) aufweist und die Speichereinheit Befehle und/oder Parameter zur Schätzung der Kanülendruckdifferenz speichert.

**11.** Herzunterstützungssystem nach einem der Ansprüche 9 bis 10, wobei die Steuereinheit mit dem Druckerfassungssystem verbunden ist, so dass mittels des Druckerfassungssystems erfasste Daten an die Steuereinheit weiterleitbar sind.

**12.** Herzunterstützungssystem nach einem der Ansprüche 9 bis 11, wobei die Steuereinheit derart ausgebildet ist, dass zur Schätzung der Kanülendruckdifferenz mindestens eine mit der Pumpe erfasste Größe herangezogen wird.

**13.** Herzunterstützungssystem nach Anspruch 12, wobei die Steuereinheit derart ausgebildet ist, dass die Kanülendruckdifferenz in Abhängigkeit eines durch die Pumpe fließenden Volumenstroms ermittelt wird, wobei die Steuereinheit derart ausgebildet ist, dass der Volumenstrom vorzugsweise anhand der Pumpendruckdifferenz ermittelt wird.

**14.** Herzunterstützungssystem nach einem der Ansprüche 9 bis 13, wobei die Pumpendruckdifferenz mittels einer Verschiebung eines Pumpenrotors ermittelt wird.

**15.** Herzunterstützungssystem nach einem der Ansprüche 9 bis 14, wobei das Druckerfassungssystem einen Drucksensor (38) am Pumpeneinlass und/oder einen Drucksensor (40) am Pumpenauslass und/oder einen Differenzdrucksensor in der Pumpe umfasst.

**Claims**

**1.** A method for determining an outlet pressure ($p_{ao}$), in particular an aortic pressure, prevailing at an outlet of a heart assist system (1; 20) comprising a pump and a cannula connected thereto, wherein the method comprises the following steps:

- determining an inlet pressure ($p_v$) which prevails at the inlet of the heart assist system;
- determining a pump pressure difference ($\Delta p$) between the pump inlet and the pump outlet of the pump of the heart assist system;
- estimating a pressure difference between the inlet and the outlet of the heart assist system based on the pump pressure difference ($\Delta p$) and an estimated cannula pressure difference ($\Delta p_c$);
- determining the outlet pressure from the inlet pressure and the pressure difference.

**2.** The method according to claim 1, wherein the inlet pressure is determined using a pressure sensor.

**3.** The method according to claim 1 or 2, wherein the determining of the pump pressure difference takes place using a current prevailing at a control coil, wherein the control coil determines a position of the pump rotor.

**4.** The method according to claim 1 or 2, wherein the pump pressure difference is detected by two pressure sensors arranged at the inlet and outlet of the pump or by a differential pressure sensor arranged in the pump.

5. The method according to claim 1, wherein the pressure difference is estimated by a non-linear function, or wherein the pressure difference is approximated by a non-linear function of a volume flow, wherein parameters of the non-linear function are determined from measurement points at which the pressure difference is known.

6. The method according to any one of the preceding claims, wherein a dynamic pressure difference term is determined by a differential equation.

7. A method according to one of the preceding claims, wherein a model of the pressure difference is determined and at least one parameter of the model is determined by way of statistical methods.

8. A method according to one of the preceding claims, wherein the pressure difference is estimated by way of a higher order polynomial and wherein the polynomial is preferably a function of a variable which is measurable by the heart assist system.

9. A heart assist system (1; 20) with a pump (2; 21), for the assistance of the heart and with a cannula (3; 50) which is connectable to the pump, wherein the pump comprises a pump inlet (22) and a pump outlet (24), and the cannula comprises a cannula inlet (52) and a cannula outlet (54), and the heart assist system comprises a pressure detection system which is configurable in a manner such that a pressure prevailing at the pump inlet is detectable and that a pump pressure difference between the pump inlet and the pump outlet is detectable, and wherein a control unit (60) is present, which is configured in a manner such that a cannula pressure difference ($\Delta p_c$) between the cannula inlet and the cannula outlet can be estimatedusing the pump pressure difference.

10. A heart assist system according to claim 9, wherein the control unit comprises a memory unit (64, 66), and the memory unit stores commands and/or parameters for estimating the cannula pressure difference.

11. A heart assist system according to one of the claims 9 to 10, wherein the control unit is connected to the pressure detection system so that data detected by the pressure detection system can be transferred to the control unit.

12. A heart assist system according to one of the claims 9 to 11, wherein the control unit is designed in a manner such that at least one variable detected by the pump is used for estimating the cannula pressure difference.

13. A heart assist system according to claim 12, wherein the control unit is designed in a manner such that the cannula pressure difference is determined in dependence on a volume flow flowing through the pump, wherein the control unit is designed such that the volume flow is preferably determined on the basis of the pump pressure difference.

14. A heart assist system according to one of claims 9 to 13, wherein the pump pressure difference is determined by way of a displacement of a pump rotor.

15. A heart assist system according to one of the claims 9 to 14, wherein the pressure detection system comprises a pressure sensor (38) at the pump inlet and/or a pressure sensor (40) at the pump outlet and/or a differential pressure sensor in the pump.

**Revendications**

1. Procédé de détermination d'une pression de sortie ($p_{ao}$), notamment d'une pression aortique, régnant à une sortie d'un système d'assistance cardiaque (1 ; 20) comprenant une pompe et une canule reliée avec elle, où le procédé comprend les étapes suivantes :

- détermination d'une pression d'entrée ($p_v$) régnant à une entrée du système d'assistance cardiaque ;
- détermination d'une différence de pression ($\Delta p$) entre une entrée de pompe et une sortie de pompe de la pompe du système d'assistance cardiaque ;
- évaluation d'une différence de pression entre l'entrée et la sortie du système d'assistance cardiaque sur la base de la différence de pression ($\Delta p$) et une différence de pression de canule ($\Delta p_c$) évaluée ;
- détermination de la pression de sortie à partir de la pression d'entrée et de la différence de pression.

2. Procédé selon la revendications 1, dans lequel la pression d'entrée est déterminé à l'aide d'un capteur de pression.

3. Procédé selon la revendication 1 ou 2, dans lequel la détermination de la différence de pression s'effectue à l'aide d'un courant régnant à une bobine de commande, dans lequel la bobine de commande détermine une position d'un rotor de pompe.

**4.** Procédé selon la revendication 1 ou 2, dans lequel la différence de pression de la pompe est détectée par deux capteurs de pression qui sont disposés à l'entrée et à la sortie de la pompe ou par un capteur de différence de pression dans la pompe.

**5.** Procédé selon la revendication 1, dans lequel la différence de pression est estimée par une fonction non linéaire, ou dans lequel la différence de pression est approchée par une fonction non linéaire d'un débit volumétrique, les paramètres de la fonction non linéaire étant déterminés à partir de points de mesure où la différence de pression est connue.

**6.** Procédé selon l'une quelconque des revendications précédentes, dans lequel un terme de différence de pression dynamique est détecté par une équation différentielle.

**7.** Procédé selon l'une des revendications précédentes, dans lequel un modèle de la différence de pression est déterminé et au moins un paramètre du modèle est déterminé à l'aide de méthodes statistiques.

**8.** Procédé selon l'une des revendications précédentes, dans lequel la différence de pression est évaluée au moyen d'un polynome d'ordre élevé et dans lequel le polynome est de préférence une fonction d'une grandeur mesurable par le système d'assistance cardiaque.

**9.** Système d'assistance cardiaque (1 ; 20) doté d'une pompe (2 ; 21) pour l'assistance cardiaque et d'une canule (3 ; 50) pouvant être reliée avec la pompe, où la pompe présente entrée de pompe (22) et une sortie de pompe (24) et la canule comprend une entrée de canule (52) et une sortie de canule (54) et le système d'assistance cardiaque présente un système de détection de la pression, lequel peut être conçu de telle manière qu'une pression régnant à l'entrée de pompe est détectable et qu'une différence de pression de pompe peut être détectée entre une entrée de pompe et une sortie de pompe, et dans lequel une unité de commande (60) est présente, laquelle est conçue de telle manière qu'une différence de pression de canule ($\Delta p_c$) peut être évaluée entre l'entrée de canule et la sortie de canule à l'aide de la différence de pression de pompe.

**10.** Système d'assistance cardiaque selon revendication 9, dans lequel l'unité de commande présente une unité de mémoire (64, 66) et l'unité de mémoire stocke des ordres et/ou des paramètres permettant l'évaluation de la différence de pression de canule.

**11.** Système d'assistance cardiaque selon l'une des revendications 9 à 10, dans lequel l'unité de commande est reliée avec le système de détection de la pression de sorte que des données détectées au moyen du système de détection de la pression peuvent être transmises ultérieurement à l'unité de commande.

**12.** Système d'assistance cardiaque selon l'une des revendications 9 à 11, dans lequel l'unité de commande est conçue de telle manière qu'au moins une grandeur détectée par la pompe est employée pour l'évaluation de la différence de pression de canule.

**13.** Système d'assistance cardiaque selon la revendication 12, dans lequel l'unité de commande est conçue de telle manière que la différence de pression de canule est déterminée en fonction d'un débit volumique traversant la pompe, dans lequel l'unité de commande est conçue de telle manière que le débit volumique est de préférence déterminé à l'aide de la différence de pression de pompe.

**14.** Système d'assistance cardiaque selon l'une des revendications 9 à 13, dans lequel la différence de pression de pompe est de préférence déterminée au moyen d'un décalage de rotor de pompe.

**15.** Système d'assistance cardiaque selon l'une des revendications 9 à 14, dans lequel le système de détection de la pression comprend un capteur de pression (38) à l'entrée de pompe, et/ou un capteur de pression (40) à la sortie de pompe, et/ou un capteur de différence de pression dans la pompe.

## FIG 1

FIG 2A

FIG 2B

## FIG 3

110            120        100       130

## FIG 4

120

122              124

## FIG 5

310        320        300       360

330

350

340

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20030045772 A1
- WO 2013021014 A
- WO 2012150045 A
- WO 2012149946 A
- WO 2011054545 A
- WO 02066837 A
- WO 2012051454 A
- WO 2012024493 A